# EUROPEAN PATENT SPECIFICATION

(11) **EP 2 540 698 B1**
(45) Date of publication and mention of the grant of the patent: **05.04.2017**
(21) Application number: 12179825.0
(22) Date of filing: 17.11.2009
(51) Int. Cl.: C07C 209/00, C07C 227/16, C07C 231/12, C07D 263/22

(54) **Method for making phenylethylamine compounds**
Verfahren zur Herstellung von Phenylethylaminverbindungen
Procédé pour la fabrication de composés phényléthylamines

(30) Priority: 19.11.2008 US 273825
(43) Date of publication of application: 02.01.2013
(62) Divisional of application: 09756166.6
(73) Proprietor: Johnson Matthey PLC, London EC4A 4AB (GB)
(72) Inventor: Fishbein, Paul, Loren, Cherry Hill, New Jersey 08003 (US); Mencel, James, North Wales, Pennsylvania 19454 (US)
(74) Representative: Whitcombe, Nicole Jane

(56) References cited:
- US-B1- 6 399 828
- YONGEUN KIM ET AL.: "Asymmetric Formal Synthesis of (-)-Formoterol and (-)-Tamsulosin", HETEROCYCLES, vol. 71, 15 June 2007 (2007-06-15), pages 2243-2248, XP008118699, Japan
- FRANZ EFFENBERGER ET AL.: "Stereoselctive Synthesis of (S)-3.4-Methylenedioxyamphetamines from (R)-Cyanohydrins", CHEMISTRY A EUROPEAN JOURNAL, vol. 3, no. 8, 1997, pages 1370-1374, XP002567952, Weinheim DE

## Description

### FIELD OF THE INVENTION

The present invention relates to a method for making phenylethylamine compounds, and a group of novel phenylethylamine intermediates.

### BACKGROUND OF THE INVENTION

The present invention relates to a method for making phenylethylamine compounds, and a group of novel phenylethylamine intermediates.

Amphetamine is commonly considered to be the most important phenylethylamine, and to date amphetamine has been made by many different methods. Only a few of these methods have been practiced commercially, and each of these methods has its disadvantages.

The classic method used to make amphetamines is the reduction of a derivative of norephedrine or norpseudoephedrine. The advantage this method has is that the resulting stereochemistry of the amphetamine product is controlled by the stereochemistry of the starting material. However, the reduction of norephedrine is difficult and can give rise to by-products that are difficult to remove. Various derivatives of norephedrine have been prepared to facilitate the reduction. For example, converting norephedrine to the benzylic chloride (Noggle F T, DeRuiter J, Clark C R, J. Chem. Sci. 1997, 25, 38-42) improves the ease of the reduction and has been known for some time, however the preparation of the chloride requires the use of hazardous, corrosive reagents. In another approach (US 6,399,828) norephedrine in converted to the benzylic acetate, which also improves the ease of the reduction, however the preparation of the acetate gives rise to undesired acetamide by-products.

Many of the other methods that are used to make amphetamines start with phenyl-2-propanone, a highly flammable liquid, which is a Schedule II controlled substance, i.e. its use is strictly regulated. The Schedule II designation and flammability impose a need for special storage and handling. These limitations complicate and add cost to the use of phenyl 2-propanone as a starting material. The condensation between phenylacetone and ammonia or an ammonia derivative typically uses Raney Nickel to reduce the resulting imine derivative (Haskelberg L, J. Am. Chem. Soc., 1948, 70, 2811-2812). This approach produces various by-products and impurities, while Raney Nickel itself is pyrophoric and therefore requires careful handling. The Leukart-Wallach reaction between formamide or ammonium formate and phenylacetone requires high temperatures and produces many by-products and impurities (Moore M L in "The Leukart Reaction", Adams R, Bachman, W E, Blatt A H, Fieser L F, Johnson J R, Synder H R, Eds. Organic Reactions, Vol. V; Wiley & Sons: New York, 1949, 301-330; Sinnema A, Verweij A M A, Bull. Narcotics, 1981, 33, 37-54). Additionally, the reaction times of methods progressing via this route are variable, as is the complexity of the necessary workup of impure product.

Amphetamines may also be manufactured from phenylalanine (Repke D B, Bates D K, Ferguson W J, J. Pharm. Sci., 1978, 67, 1167-1168), which enables the stereochemistry of the amphetamine product to be controlled by the stereochemistry of the amino acid used. These syntheses, however, involve many steps and require the use of hydride reagents or multiple catalytic hydrogenations, some of which use Raney Nickel thereby incurring the disadvantages mentioned above.

Dextroamphetamine may be obtained by the resolution of the racemate through the tartrate salt (US 6,399,828). However, this is a labour-intensive, low yield process.

The hydrogenation of 1,3-oxazolidin-2-ones has been reported in Effenburger et al (Chem. Eur. J., 1997, 3(8), 1370). The method involves the use of triethylamine which must be later removed in high vacuo before an amphetamine hydrochloride salt can be precipitated. The authors appear to believe that the presence of triethylamine is essential in order to facilitate the hydrogenation reaction. The inventors, however, believe that the addition of triethylamine prevents the hydrogenation reaction being commercially viable as the separation of the amine from the amphetamine would be extremely difficult and expensive on a commercial scale.

Kim et al (Heterocycles, 2007, 71(10), 2243) describes the asymmetric formal synthesis of (-)-formoterol and (-)-tamsulosin.

### SUMMARY OF THE INVENTION

We have developed a method for the manufacture of phenylethylamines, including amphetamine, that avoids the use of bases, avoids the use of toxic and dangerous reagents, requires mild reaction conditions, produces essentially no by-products or impurities in the final products thereby eliminating the need for separate purification steps, is short, does not use controlled substances, is very robust and is suited to large-scale manufacture. This method also takes advantage of the innate stereochemistry of the starting materials, thereby eliminating the need for resolution steps.

### DETAILED DESCRIPTION OF THE INVENTION

According to a first aspect, the invention provides a method suitable for the large-scale manufacture of a pharmaceutically acceptable salt of a phenylethylamine of formula B: 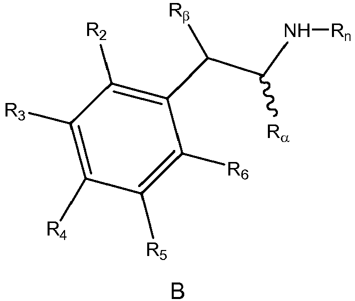 wherein R₂, R₃, R₄, R₅, R₆, R_{α}, R_{β} and Rₙ are each independently selected from hydrogen, C₁₋₂₀-alkyl, -C(O)R' wherein R' is a C₁₋₂₀-alkyl or C₆₋₂₀-aryl, C₆₋₂₀-aryl,-C(O)NR'R" wherein R' and R" are independently selected from C₁₋₂₀-alkyl or C₆₋₂₀-aryl, or Rₙ is -CH₂Ph;
wherein "alkyl" represents a cyclic, branched or straight chain saturated hydrocarbon group said method comprising:
(a) the reduction of a compound of formula A in at least one solvent in the absence of acid or base: 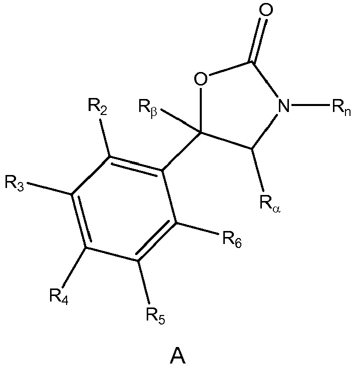 wherein R₂, R₃, R₄, R₅, R₆, R_{α}, R_{β} and Rₙ are as defined above;
(b) once the reduction is complete, the reaction mixture is heated if required and the catalyst removed; and
(c) adding an acid to the reaction mixture to form the pharmaceutically acceptable salt of the phenylethylamine of formula B.

The compounds of formulae A and B can contain one or more chiral centres. The present invention therefore relates to all enantiomers, diastereomers and mixtures thereof.

As used herein, the term "alkyl" refers to a cyclic, branched or straight chain saturated hydrocarbon group preferably having 1 to 20 carbon atoms. Examples of C₁-C₂₀ alkyl groups include, but are not limited to, methyl, ethyl, n-propyl, isopropyl, n-butyl, t-butyl, n-pentyl, cyclopentyl, n-hexyl, cyclohexyl and the like.

The term "acyl" refers to a group of the formula -C(O)R' preferably having 1 to 20 carbon atoms, wherein R' is a substituent selected from alkyl or aryl group.

The term "aryl" refers to an aromatic carbocyclic group preferably having 6 to 20 carbon atoms. The aryl group may have a single ring or multiple condensed rings. Examples of C₆-C₂₀ aryl groups include, but are not limited to, phenyl, naphthyl, anthracenyl and the like. Unless otherwise specified, the aryl group may be attached at any suitable carbon atom.

When Rₙ is -CH₂Ph (i.e. benzyl), a debenzylation reaction may occur prior to, concomitantly with or after the reduction of the compound of formula A.

The term "amido" refers to a group of the formula -C(O)NR'R" preferably having 1 to 20 carbon atoms, wherein R' and R" are substituents independently selected from alkyl or aryl groups.

Step (a) of the method of the present invention is carried out in the absence of base. This offers the processing advantage in that the compounds of formula B can be isolated as the free amine by simple solvent minimisation.

In one embodiment, the reduction is carried out using gaseous hydrogen and a catalyst. Conveniently, the reduction will be carried out using gaseous hydrogen at atmospheric pressure or above (e.g. 5-25 psig). In a preferred embodiment, the catalyst is palladium on carbon. However, there are alternative means of carrying out the reducing step. Such means may include carrying out transfer hydrogenolysis using hydrogen donor agents such as readily dehydrogenatable hydrocarbons, (e.g. methyl cyclohexene), formic acid, ammonium or potassium formate, and hydrazine (Brieger G, Nestrick T, J. Chem. Rev., 1974, 74, 567-580). The skilled person will appreciate that other means of carrying out the reduction may also be used.

The reduction is commonly carried out at a temperature of from about 0°C to about 30°C, conveniently from about 20°C to about 25°C.

Step (a) of the method of the present invention is carried out in the absence of acid. The advantage of carrying out the reaction in such a way is that the phenylethylamine of formula B may be prepared as the free base. If desired, the phenylethylamine of formula B as the free base may then be treated directly with an acid to prepare a salt. In this instance, there is no requirement for an exchange of salts to be performed to obtain a desired pharmaceutically acceptable salt once the reduction reaction has taken place. Moreover, if the salt exchange requires the intermediate preparation of the phenylethylamine of formula B as a free base, a solvent switch may also be necessary if the solvent or solvent mixtures present are not amenable to the freebasing operation. When carried out in the absence of an acid, therefore, the method of the present invention may additionally avoid the necessity of a solvent switch.

A variety of solvents or solvent mixtures may be used with the method of the present invention, including those comprising alcohols and/or aromatic hydrocarbons. Preferably, the alcoholic solvent is methanol, ethanol, butanol or SDA-3A (a solvent constituting 95% ethanol and 5% methanol). More preferably, the alcohol is butanol or SDA-3A. When the solvent is an aromatic hydrocarbon, it is preferably, benzene or toluene.

As explained above the method disclosed herein may be used to manufacture a range of phenylethylamines. Such phenylethylamines include amphetamine, i.e. when the R groups of formulae A and B are defined as follows: R₂, R₃, R₄, R₅, R₆, R_{β} and Rₙ are hydrogen, and R_{α} is a -CH₃ group, and methamphetamine, i.e. when the R groups of formulae A and B are defined as follows R₂, R₃, R₄, R₅, R₆ and R_{β} are hydrogen, and R_{α} and Rₙ are -CH₃ groups. More complex phenylethylamines may also be manufactured, such as those when the R groups of formulae A and B are defined as follows: R₂, R₃, R₄, R₅, R₆ and R_{β} are hydrogen, and Rₙ is an aryl group.

The method of the present invention further comprises the preparation of a pharmaceutically acceptable salt of the compound of formula B. By "pharmaceutically acceptable salt" we mean a therapeutically active, non-toxic salt which may be derived from organic or inorganic counterions. Pharmaceutically acceptable salts are well known in the art, for example, see "Pharmaceutical Sciences: The Science and Practice of Pharmacy (Remington: The Science and Practice of Pharmacy)", Lippincott Williams and Wilkins. Examples of pharmaceutically acceptable salts include, but are not limited to, hydrochloride, hydrobromide, sulphate, saccharate, aspartate, mesylate, dimesylate and the like.

In step (c), the pharmaceutically acceptable salt is prepared by the direct addition of the acid to the reaction mixture once the reduction is complete. In this case, in step (b), once the reduction is complete, the reaction mixture may be heated if required and the catalyst removed (e.g. by filtering through Celite). An aqueous acid solution is added (e.g. aqueous sulphuric acid, aqueous saccharic acid solution or aqueous aspartic acid solution) and excess water removed. Preferably, the pharmaceutically acceptable salt of the compound of formula B is recovered, for example, by filtering or decanting, and optionally dried.

When the compound of formula B is amphetamine, the pharmaceutically acceptable salt is preferably sulphate or aspartate. When the compound of formula B is methamphetamine, the pharmaceutically acceptable salt is preferably hydrochloride. When the compound of formula B is dextroamphetamine, the pharmaceutically acceptable salt is preferably sulphate, aspartate or saccharate and more preferably, sulfate.

In order that the invention may be more fully understood, the following Examples are provided by way of illustration only:

### Example 1

### Dextroamphetamine

### (not according to the invention)

A mixture of 6.00 g (33.9 mmol) (4S,5R)-(-)-4-methyl-5-phenyl-2-oxazolidinone, 0.34 g 10% palladium-on-carbon (50% water wet), and 60 mL SDA-3A (a solvent constituting 95% Ethanol and 5% Methanol) was stirred under a hydrogen filled balloon at ambient temperature (approximately 20°C) until no more oxazolidinone was detected by HPLC, 4 h. The reaction mixture was then filtered through a pad of Celite to remove the catalyst. For the purposes of isolating the free base, a filtered reaction mixture was carefully concentrated under reduced pressure to leave 4.47 g of a crystalline semi-solid, which was found to be a mixture of 96% dextroamphetamine and 4% ethanol by 1H NMR. Some dextroamphetamine was detected in the distillate. Yield = 94%. 400 MHz 1 H NMR (CDCl₃) 7.34-7.20 (m, 5), 3.22-3.17 (m, 1), 2.76-2.72 (d of d, J = 13.2 Hz, J' = 5.4 Hz, 1), 2.57-2.52 (d of d, J = 13.2 Hz, J' = 8.1 Hz, 1), 1.16-1.14 (d, J = 6.3 Hz, 3).

### Example 2

### Amphetamine

### (not according to the invention)

A mixture of 6.00 g (33.9 mmol) rac-cis-4-methyl-5-phenyl-2-oxazolidinone, 0.34 g 10% palladium-on-carbon (50% water wet), and 60 mL SDA-3A was stirred under a hydrogen filled balloon at ambient temperature until no more oxazolidinone was detected by HPLC (7 h). The reaction mixture was then filtered through a pad of Celite to remove the catalyst.

### Example 3

### Methamphetamine

### (not according to the invention)

A mixture of 1.00 g (5.23 mmol) (4S,5R)-3,4-dimethyl-5-phenyl-2-oxazolidinone, 0.0530 g 10% palladium-on-carbon (50% water wet), and 10 mL SDA-3A was stirred under a hydrogen filled balloon at ambient temperature overnight. The reaction had no detectable oxazolidinone by HPLC in the morning.

### Example 4

### N-Acetyl-d-amphetamine

### (not according to the invention)

A mixture of 1.00 g (4.56 mmol) (4S,5R)-3-Acetyl-4-methyl-5-phenyl-2-oxazolidinone, 0.0460 g 10% palladium-on-carbon (51% water wet), and 10 mL SDA-3A was stirred under a hydrogen filled balloon at ambient temperature overnight. The reaction had no detectable oxazolidinone by HPLC in the morning. The reaction mixture was then filtered through a pad of Celite to remove the catalyst. The filtrate was carefully concentrated under reduced pressure to leave 0.98 g of a liquid, which proved to be a mixture of 81%, N-acetyl-d-amphetamine, 19% ethanol, and 1% methanol. Yield = 98%. 400 MHz 1 H NMR (CDCl₃) 7.34-7.18 (m, 5), 4.30-4.26 (m, 1), 2.87-2.83 (d of d, J = 13.5 Hz, J' = 5.7 Hz, 1), 2.76-2.71 (d of d, J = 13.5 Hz, J' = 7.2 Hz, 1), 1.95 (s, 3), 1.13-1.11 (d, J = 6.7 Hz, 3).

### Example 5

### Dextroamphetamine Sulfate

The method of Example 1 was followed until the catalyst was filtered away whereupon a total of 7 mL 25% H₂SO₄ (aq) was added dropwise to the filtrate over 3 min. After cooling in an ice/water bath for 15 min, the mixture was filtered to collect the dextroamphetamine sulfate using cold SDA-3A as a flask and cake rinse. After drying, a total of 5.79g of dextroamphetamine sulfate was obtained, 93% yield. This material was assayed at 100% by HPLC.

### Example 6

### Dextroamphetamine Saccharate

A mixture of (4S,5R)-4-Methyl-5-phenyl-2-oxazolidinone (20.00 g, 112.9 mmol), 210 mL 1-Butanol, and 1.14 g Water wet 10% Palladium-on-Carbon was made. The Palladium-on-Carbon was about 50% Water wet. The mixture was hydrogenated at 20-25 °C, 20-25 psig until there was not more than 0.09% remaining Oxazolidinone when normalized to the Dextroamphetamine by HPLC. This took approximately 5.5 h. The reaction mixture was heated to 30-35 °C and filtered through Celite to remove the catalyst using 42 mL 1-Butanol as a flask and cake rinse. An aqueous Saccharic Acid solution (132 mL, 0.09 g/mL, 56.5 mmol) was then added to the filtrate. The resulting solution was then concentrated under reduced pressure at a temperature of not more than 65 °C to a volume of about 180 mL to remove Water by azeotropic distillation. A total of 105 mL 1-Butanol was added and the distillation repeated. The addition of 1-Butanol and subsequent distillation was repeated until the Water content was less than or equal to 2.0%. To the resulting solution was added 358 mL Acetone, the mixture cooled to -5-0 °C, and the Dextroamphetamine Saccharate was then collected by vacuum filtration. After drying, 22.93 g (85%) of Dextroamphetamine Saccharate was obtained.

### Example 7

### Amphetamine Aspartate

A mixture of rac-cis-4-Methyl-5-phenyl-2-oxazolidinone (38.00 g, 214.4 mmol), 380 mL 1-Butanol, and 2.15 g Water wet 10% Palladium-on-Carbon was made. The Palladium-on-Carbon was about 50% Water wet. The mixture was hydrogenated at 20-25 °C, 20-25 psig until there was not more than 0.09% remaining Oxazolidinone when normalized to the Amphetamine by HPLC. The mixture was then heated to 30-35 °C and filtered through Celite using 1-Butanol as a flask and cake rinse. The filtrate was heated to 40-50 °C and added to an aqueous solution of Aspartic Acid (28.50 g, 214.1 mmol, dissolved in 410 mL Water) also at 40-50 °C. The resulting mixture was then polish filtered at 55-60 °C. The filtrate was vacuum distilled at less than 65 °C to bring the Water content to not more than 1.0%. The final target volume was 431 mL. The distillation was repeated several times in order to achieve this by adding 1-Butanol and then distilling. Once the desired Water content was achieved, the target volume was attained by either continuing the distillation or adding more 1-Butanol. The resulting mixture was cooled to 0-5 °C and 232 mL SDA-3A was added. The Amphetamine Aspartate was then collected and dried to give 47.72 g (83%).

### Example 8

### Amphetamine Sulfate

A mixture of rac-cis-4-Methyl-5-phenyl-2-oxazolidinone (40.00 g, 225.7 mmol), 400 mL SDA-3A, and 2.27 g Water wet 10% Palladium-on-Carbon was made. The Palladium-on-Carbon was about 50% Water wet. The mixture was hydrogenated at 20-25 °C, 5-10 psig until there was not more than 0.09% remaining Oxazolidinone when normalized to the Amphetamine by HPLC. The mixture was then filtered through Celite to remove the catalyst and 37.53 mL Water was added. The temperature was brought to 68-75 °C and 44.28 g 25% Sulfuric Acid (aq) was added maintaining 68-75 °C. The mixture was then cooled to 0-5 °C and the Amphetamine Sulfate was then collected to give 33.35 g (80%).

## Claims

1. A method suitable for the large-scale manufacture of a pharmaceutically acceptable salt of a phenylethylamine of formula B: 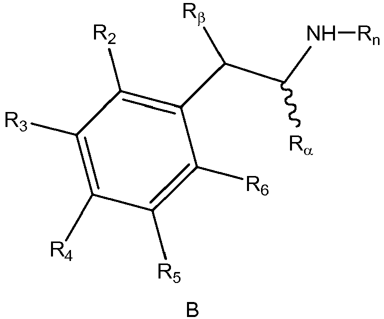 wherein R₂, R₃, R₄, R₅, R₆, R_{α}, R_{β} and Rₙ are each independently selected from hydrogen, C₁₋₂₀-alkyl, -C(O)R' wherein R' is a C₁₋₂₀-alkyl or C₆₋₂₀-aryl, C₆₋₂₀-aryl, -C(O)NR'R" wherein R' and R" are independently selected from C₁₋₂₀-alkyl or C₆₋₂₀-aryl, or Rₙ is -CH₂Ph;
wherein "alkyl" represents a cyclic, branched or straight chain saturated hydrocarbon group; said method comprising:
(a) the reduction of a compound of formula A in at least one solvent in the absence of acid or base: 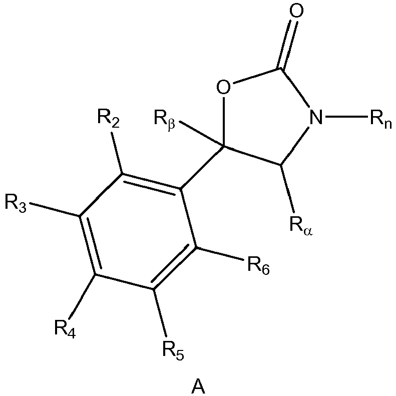 wherein R₂, R₃, R₄, R₅, R₆, R_{α}, R_{β} and Rₙ are as defined above;
(b) once the reduction is complete, the reaction mixture is heated if required and the catalyst removed; and
(c) adding an acid to the reaction mixture to form the pharmaceutically acceptable salt of the phenylethylamine of formula B.

2. A method according to claim 1, wherein the reduction is carried out using (a) gaseous hydrogen and a catalyst or (b) a hydrogen transfer agent.

3. A method according to claim 2, wherein the reduction is carried out using gaseous hydrogen at atmospheric pressure or above.

4. A method according to claim 2, wherein the catalyst is palladium on carbon.

5. A method according to any one of the preceding claims, wherein the reduction is carried out at a temperature of from about 0°C to about 30°C.

6. A method according to claim 5, wherein the temperature is from about 20°C to about 25°C.

7. A method according to any one of the preceding claims, wherein the reduction is carried out in the presence of at least one solvent selected from the group consisting of alcohols and aromatic hydrocarbons.

8. A method according to claim 7, wherein the alcohol is selected from the group consisting of methanol, ethanol, butanol and SDA-3A (a solvent constituting 95% ethanol and 5% methanol).

9. A method according to claim 7, wherein the aromatic hydrocarbon is selected from the group consisting of benzene and toluene.

10. A method according to any one of the preceding claims, wherein R₂, R₃, R₄, R₅, R₆, R_{β} and Rₙ are hydrogen, and R_{α} is a -CH₃ group.

11. A method according to any one of claims 1 to 9, wherein R₂, R₃, R₄, R₅, R₆ and R_{β} are hydrogen, and R_{α} and Rₙ are -CH₃ groups.

12. A method according to any one of claims 1 to 9, wherein R₂, R₃, R₄, R₅, R₆ and R_{β} are hydrogen, and Rₙ is an aryl group.

13. A method according to claim 1, wherein the pharmaceutically acceptable salt of the phenylethylamine of formula B is selected from the group consisting of hydrochloride, hydrobromide, sulphate, saccharate, aspartate, mesylate and dimesylate.

14. A method according to claim 1, wherein an aqueous acid solution is added and excess water removed.

## Patentansprüche

1. Verfahren, das sich zur Herstellung eines pharmazeutisch unbedenklichen Salzes eines Phenylethylamins der Formel B in großem Maßstab eignet 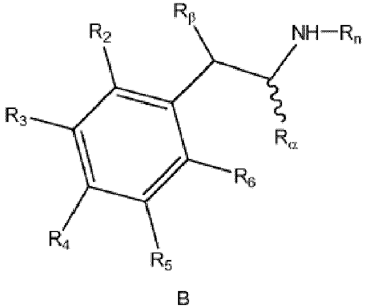 wobei R₂, R₃, R₄, R₅, R₆, R_{α}, R_{β} und Rₙ jeweils auf unabhängige Weise aus Wasserstoff, C₁₋₂₀-Alkyl, -C(O)R', wobei R' für ein C₁₋₂₀-Alkyl oder C₆₋₂₀-Aryl steht, C₆₋₂₀-Aryl, -C(O)NR'R", wobei R' und R" in jeweils unabhängiger Auswahl für C₁₋₂₀-Alkyl oder C₆₋₂₀-Aryl steht, ausgewählt sind, oder Rₙ gleich -CH₂Ph ist;
wobei "Alkyl" für eine zyklische, verzweigte oder geradkettige Kohlenwasserstoffgruppe gesättigter Art steht, wobei das Verfahren Folgendes umfasst:
(a) Reduktion einer Verbindung der Formel A in mindestens einem Lösungsmittel in Abwesenheit von Säuren oder Basen: 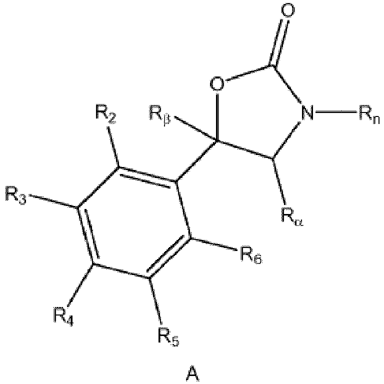 wobei R₂, R₃, R₄, R₅, R₆, R_{α}, R_{β} und Rₙ den obigen Begriffsbestimmungen entsprechen;
(b) nach dem Abschluss der Reduktion wird die Reaktionsmischung erforderlichenfalls erwärmt und der Katalysator entfernt; und
(c) Hinzufügen einer Säure zu der Reaktionsmischung, um das pharmazeutisch unbedenkliche Salz des Phenylethylamins nach Formel B zu bilden.

2. Verfahren nach Anspruch 1, wobei die Reduktion unter Verwendung (a) von gasförmigem Wasserstoff und eines Katalysators oder (b) eines Wasserstoffübertragungsmittels durchgeführt wird.

3. Verfahren nach Anspruch 2, wobei die Reduktion unter Verwendung gasförmigen Wasserstoffs bei Atmosphärendruck oder höherem Druck durchgeführt wird.

4. Verfahren nach Anspruch 2, wobei es sich bei dem Katalysator um kohlenstoffgeträgertes Palladium handelt.

5. Verfahren nach einem beliebigen der vorhergehenden Ansprüche, wobei die Reduktion bei einer Temperatur von ungefähr 0 °C bis ungefähr 30 °C durchgeführt wird.

6. Verfahren nach Anspruch 5, wobei die Temperatur ungefähr 20 °C bis ungefähr 25 °C beträgt.

7. Verfahren nach einem beliebigen der vorhergehenden Ansprüche, wobei die Reduktion in Gegenwart mindestens eines Lösungsmittels durchgeführt wird, das aus der Gruppe ausgewählt ist, welche aus Alkoholen und aromatischen Kohlenwasserstoffen besteht.

8. Verfahren nach Anspruch 7, wobei der Alkohol aus der Gruppe ausgewählt ist, welche aus Methanol, Ethanol, Butanol und SDA-3A (einem Lösungsmittel aus 95 % Ethanol und 5 % Methanol) besteht.

9. Verfahren nach Anspruch 7, wobei der aromatische Kohlenwasserstoff aus der Gruppe ausgewählt ist, die aus Benzol oder Toluol besteht.

10. Verfahren nach einem beliebigen der vorhergehenden Ansprüche, wobei R₂, R₃, R₄, R₅, R₆, R_{β} und Rₙ gleich Wasserstoff sind und R_{α} für eine -CH₃-Gruppe steht.

11. Verfahren nach einem beliebigen der Ansprüche 1 bis 9, wobei R₂, R₃, R₄, R₅, R₆ und R_{β} gleich Wasserstoff sind und R_{α} und Rₙ für -CH₃-Gruppen stehen.

12. Verfahren nach einem beliebigen der Ansprüche 1 bis 9, wobei R₂, R₃, R₄, R₅, R₆ und R_{β} gleich Wasserstoff sind und Rₙ für eine Arylgruppe steht.

13. Verfahren nach Anspruch 1, wobei das pharmazeutisch unbedenkliche Salz des Phenylethylamins der Formel B aus der Gruppe ausgewählt ist, die aus Hydrochlorid, Hydrobromid, Sulfat, Saccharat, Aspartat, Mesylat und Dimesylat besteht.

14. Verfahren nach Anspruch 1, wobei eine wässrige saure Lösung zugesetzt wird und überschüssiges Wasser entfernt wird.

## Revendications

1. Procédé approprié à la fabrication à grande échelle d'un sel pharmaceutiquement acceptable d'une phényléthylamine de formule B : 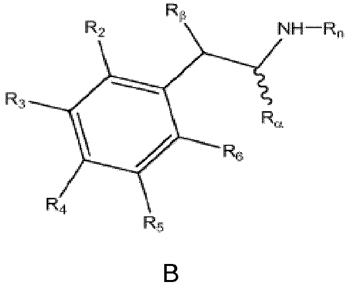 dans laquelle R₂, R₃, R₄, R₅, R₆, R_{α}, R_{β} et Rₙ sont chacun indépendamment choisis parmi un atome d'hydrogène, un alkyle en C₁₋₂₀, -C(O)R' dans lequel R' est un alkyle en C₁₋₂₀ ou un aryle en C₆₋₂₀, un aryle en C₆₋₂₀, -C(O)NR'R" dans lequel R' et R" sont indépendamment choisis parmi un alkyle en C₁₋₂₀ ou un aryle en C₆₋₂₀, ou Rₙ est -CH₂Ph ; « alkyle » représentant un groupe hydrocarboné saturé cyclique, ramifié ou linéaire ;
ledit procédé comprenant :
(a) la réduction d'un composé de formule A dans au moins un solvant en l'absence d'acide ou de base : 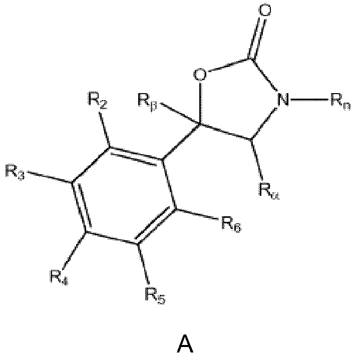 dans laquelle R₂, R₃, R₄, R₅, R₆, R_{α}, R_{β} et Rₙ sont tels que définis ci-dessus ;
(b) une fois la réduction accomplie, le chauffage du mélange réactionnel si nécessaire et la séparation du catalyseur ; et
(c) l'addition d'un acide au mélange réactionnel pour former le sel pharmaceutiquement acceptable de la phényléthylamine de formule B.

2. Procédé selon la revendication 1, dans lequel la réduction est réalisée en utilisant (a) de l'hydrogène gazeux et un catalyseur ou (b) un agent de transfert d'hydrogène.

3. Procédé selon la revendication 2, dans lequel la réduction est réalisée en utilisant de l'hydrogène gazeux à la pression atmosphérique ou à une pression supérieure.

4. Procédé selon la revendication 2, dans lequel le catalyseur est du palladium sur charbon.

5. Procédé selon l'une quelconque des revendications précédentes, dans lequel la réduction est réalisée à une température d'environ 0 °C à environ 30 °C.

6. Procédé selon la revendication 5, dans lequel la température est d'environ 20 °C à environ 25 °C.

7. Procédé selon l'une quelconque des revendications précédentes, dans lequel la réduction est réalisée en présence d'au moins un solvant choisi dans le groupe constitué par des alcools et des hydrocarbures aromatiques.

8. Procédé selon la revendication 7, dans lequel l'alcool est choisi dans le groupe constitué par le méthanol, l'éthanol, le butanol et le SDA-3A (un solvant constitué à 95 % d'éthanol et à 5 % de méthanol).

9. Procédé selon la revendication 7, dans lequel l'hydrocarbure aromatique est choisi dans le groupe constitué par le benzène et le toluène.

10. Procédé selon l'une quelconque des revendications précédentes, dans lequel R₂, R₃, R₄, R₅, R₆, R_{β} et Rₙ sont un atome d'hydrogène, et R_{α} est un groupe -CH₃.

11. Procédé selon l'une quelconque des revendications 1 à 9, dans lequel R₂, R₃, R₄, R₅, R₆ et R_{β} sont un atome d'hydrogène, et R_{α} et Rₙ sont des groupes -CH₃.

12. Procédé selon l'une quelconque des revendications 1 à 9, dans lequel R₂, R₃, R₄, R₅, R₆ et R_{β} sont un atome d'hydrogène, et Rₙ est un groupe aryle.

13. Procédé selon la revendication 1, dans lequel le sel pharmaceutiquement acceptable de la phényléthylamine de formule B est choisi dans le groupe constitué par le chlorhydrate, le bromhydrate, le sulfate, le saccharate, l'aspartate, le mésylate et le dimésylate.

14. Procédé selon la revendication 1, dans lequel une solution aqueuse acide est ajoutée et l'excès d'eau éliminé.
